# EUROPEAN PATENT APPLICATION

(11) **EP 1 298 438 A1**
(43) Date of publication of application: **02.04.2003**
(21) Application number: 01938686.1
(22) Date of filing: 15.06.2001
(51) Int. Cl.: G01N 33/543, G01N 33/58, G01N 33/53

(54) **INSOLUBLE CARRIER PARTICLE NEPHELOMETRIC IMMUNOASSAY REAGENT**

(30) Priority: 30.06.2000 JP 2000198831
(71) Applicant: KYOWA MEDEX CO., LTD., Tokyo 104-0042 (JP)
(72) Inventor: SHIGENOBU, Kayoko, Kyowa Medex Co., Ltd., Sunto-gun, Shizuoka 411-0932 (JP); OGURI, Kazuhito, Kyowa Medex Co., Ltd., Sunto-gun, Shizuoka 411-0932 (JP)
(74) Representative: Harding, Charles Thomas
(86) International application number: JP0105115
(87) International publication number: WO02003068

(57) **Abstract**

The present invention provides: a reagent and a kit for an insoluble carrier particle nephelometric immunoassay which stabilize the agglutination reaction by suppressing the action of blood plasma components that are involved in the agglutination reaction of insoluble carrier particles such as latex and affect the values to be determined, to provide the stable absorbances of the reaction solutions and the accurate determination results; and a method of an insoluble carrier particle nephelometric immunoassay utilizing said reagent or kit. An antigen or an antibody is carried on an insoluble carrier particle in the presence or absence of a buffer solution comprising a compound having within its molecule a group shown below such as bicine, tricine, then an antibody- or antigen-sensitized insoluble carrier particle suspension is allowed to contact a sample in the presence of the above mentioned buffer solution to cause the immune agglutination reaction, and the turbidity generated by the insoluble carrier particle agglutination reaction is measured to determine the antigen or antibody in the sample. 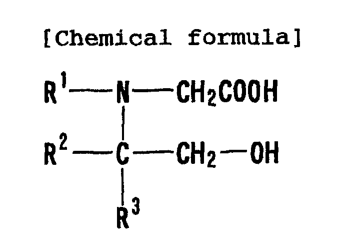 wherein R¹, R² and R³ may be the same or different, and independently represent a hydrogen atom, a hydroxyalkyl group or the like.

## Description

### Technical Field

The present invention relates to a reagent for a nephelometric immunoassay comprising insoluble carrier particles such as latex, a method of a nephelometric immunoassay using insoluble carrier particles and a kit for a nephelometric immunoassay comprising insoluble carrier particles. The present invention more specifically relates to a reagent for a nephelometric immunoassay comprising insoluble carrier particles that stabilize the absorbance and can suppress the action of blood plasma components which are involved in a reaction and affect values to be determined, a method of a nephelometric immunoassay using such insoluble carrier particles and a kit for a nephelometric immunoassay comprising such insoluble carrier particles.

### Background Art

In the field of clinical tests, latex is widely used for immunoassays that determine antigens or antibodies in the samples. For example, Japanese Published Unexamined Patent Application No.253629/98 describes a process for preparing an immunoassay reagent which comprises the following steps: antigens or antibodies are carried by polystyrene latex particles in a pH 4.0 to 6.0 buffer solution such as a pH 4.2 phosphate-citrate buffer solution; and then the buffer solution is substituted with a pH 6.5 to 9.0 buffer solution such as a pH 8.0 tris buffer solution. In said method, pro-zone phenomenon is suppressed while maintaining a high sensitivity, which results in high stability and good reproducibility in determination.

Further, Japanese Published Unexamined Patent Application No.318632/97 describes a method of a latex nephelometric immunoas say which comprises the following steps: mixing a sample and a latex suspension carrying an antigen or an antibody; adding a dihydric alcohol to the mixed solution; and measuring the changes in absorbance caused by the latex particle agglutination formed through the antigen-antibody reaction. In said method, determination can be carried out using the original solution without diluting a sample even when an antigen or an antibody to be determined is contained at a high concentration in the sample.

Still further, Japanese Published Unexamined Patent Application No.301632/95 describes a carrier microparticle comprising a latex particle having a carboxylate group on its surface and an immuno-reactant bound to said latex particle by a covalent bond, wherein the latex particle has a diameter of 0.1 to 0.6 µm and surface region of 8 to 35 square angstroms occupied by carboxylates; and an immunoassay reagent comprising said microparticles and a buffer solution.

As described above, latex is often used in the determination systems that employ an immune agglutination reaction, such as a latex nephelometric immunoassay. However, a latex nephelometric immunoassay has some drawbacks of unstabilization of sensitivity which is originated from the change in adsorptive activity of an antigen or an antibody to a latex carrier by reaction of a latex with plasma components co-existing in the latex suspension in the presence of heavy metal ions, or the change in the degree of latex agglutination in the reaction by release of antigens or antibodies from the latex to which they are bound. Also, in the case of the nephelometric immunoassay using a latex to which no antigens or antibodies are bound, there are problems of unstabilization of sensitivity originated from change in surface charges by binding of heavy metal ions to carboxyl groups or sulfone groups responsible for the surface charges.

An object of the present invention is to provide: a reagent and a kit for an insoluble carrier particle nephelometric immunoassay, which stabilizes the agglutination reaction and the absorbances of the reaction solutions to give the accurate results of determination, by suppressing the action of blood plasma components that are involved in the agglutination reaction of insoluble carrier particles such as latex and affect values to be determined; and a method of an insoluble carrier particle nephelometric immunoassay using said reagent or the kit.

The present inventors have made a keen study to solve the problems mentioned above and have found that the application of a buffer containing a compound having in its molecular formula a specific group to blood plasma components which unstabilize the latex agglutination reaction, i.e. multivalent metal ions present in a very small amount in the blood plasma, leads to stabilization of the charge condition on the latex surface, suppression of the release of antigens or antibodies bound to latex, and stabilization of the latex agglutination reaction, which results in more accurate determination. The present invention has thus been completed.

### Disclosure of the Invention

The present invention relates to a reagent for an insoluble carrier particle nephelometric immunoassay comprising insoluble carrier particles and a buffer containing a compound having within its molecule a group represented by [Chemical formula 1] shown below or a salt thereof (claim 1) ; the reagent for an insoluble carrier particle nephelometric immunoassay according to claim 1, wherein the above mentioned compound is represented by general formula [I] having [Chemical formula 2] shown below, wherein R¹ and R² may be the same or different, and independently represent hydrogen atom, alkyl group, hydroxyalkyl group, di(hydroxyalkyl)alkyl group, tri(hydroxyalkyl)alkyl group; carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxy-alkyl)alkyl group, or R¹ and R² form a cyclic structure with a nitrogen atom to give substituted or unsubstituted piperazinyl group, substituted or unsubstituted morpholino group, or substituted or unsubstituted piperidino group (claim 2); the reagent for an insoluble carrier particle nephelometric immunoassay according to claim 2, wherein the above mentioned compound represented by general formula [I] is a compound represented by general formula [II] having [Chemical formula 3] shown below, wherein R¹, R² and R³ may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, or tri(sulfo-hydroxy-alkyl)alkyl group (claim 3); the reagent for an insoluble carrier particle nephelometric immunoassay according to claim 3, wherein the compound represented by general formula [II] is bicine or tricine (claim 4); the reagent for an insoluble carrier particle nephelometric immunoassay according to any of claims 1 to 4, wherein the buffer is contained in such a manner that the concentration can be adjusted to 5 to 200 mmol/L (claim 5); the reagent for an insoluble carrier particle nephelometric immunoassay according to any of claims 1 to 5, wherein the insoluble carrier particles are contained in such a manner that the concentration can be adjusted to 0.005 to 5% by weight (claim 6); and the insoluble carrier particle nephelometric immunoassay reagent according to any of claims 1 to 6, wherein the insoluble carrier particle is latex (claim 7).

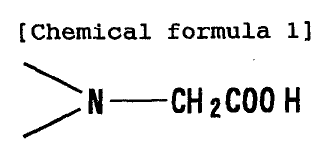

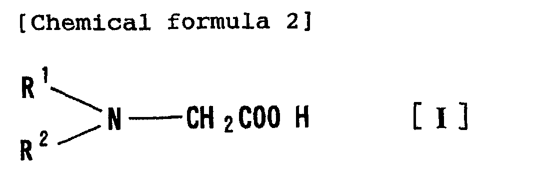

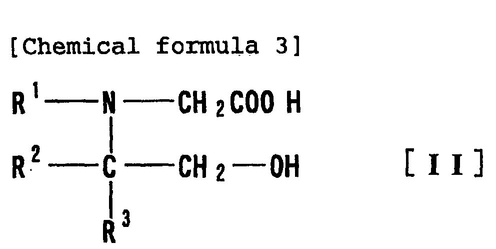

The present invention further relates to a method of an insoluble carrier particle nephelometric immunoassay using insoluble carrier particles and a buffer comprising a compound having within its molecule a group represented by the above mentioned [Chemical formula 1] or a salt thereof (claim 8); the method of an insoluble carrier particle nephelometric immunoassay according to claim 8, wherein the compound represented by general formula [I] shown by the above [Chemical formula 2], wherein R¹ and R² have the same meanings as defined above, is used (claim 9); the method of an insoluble carrier particle nephelometric immunoassay according to claim 9, wherein the compound represented by the above mentioned [Chemical formula 3] as general formula [I], wherein R¹, R² and R³ have the same meanings as defined above, is used (claim 10); the method of an insoluble carrier particle nephelometric immunoassay according to claim 10, wherein bicine or tricine is used as the compound represented by general formula [II] (claim 11); the method of an insoluble carrier particle nephelometric immunoassay according to any of claims 8 to 11, wherein an antigen or an antibody is carried by an insoluble carrier particle in the presence of a buffer and then an immune agglutination reaction is allowed to occur (claim 12); the method of an insoluble carrier particle nephelometric immunoassay according to any of claims 8 to 12, wherein an antigen or an antibody is carried by an insoluble carrier particle and then an immune agglutination reaction is allowed to occur in the presence of a buffer (claim 13); the method of an insoluble carrier particle nephelometric immunoassay according to any of claims 8 to 13, wherein the buffer is used in a buffer solution at a concentration of 5 to 200 mmol/L (claim 14); the method of an insoluble carrier particle nephelometric immunoassay according to any of claims 8 to 14, wherein the insoluble carrier particles are used in an insoluble carrier particle suspension at a concentration of 0.005 to 5% by weight (claim 15); and the method of an insoluble carrier particle nephelometric immunoassay according to any of claims 8 to 15, wherein the insoluble carrier particle is latex (claim 16).

The present invention still further relates to a kit for an insoluble carrier particle nephelometric immunoassay comprising a suspension containing insoluble carrier particles and a buffer solution containing a buffer comprising a compound having within its molecule a group represented by the above-mentioned [Chemical formula 1] or a salt thereof (claim 17); the kit for an insoluble carrier particle nephelometric immunoassay according to claim 17, wherein the above-mentioned compound is a compound represented by general formula [I] shown by the above [Chemical formula 2], wherein R¹ and R² have the same meanings as defined above (claim 18); the kit for an insoluble carrier particle nephelometric immunoassay according to claim 18, wherein the compound represented by general formula [I] mentioned above is a compound represented by general formula [II] shown by the above [Chemical formula 3], wherein R¹, R² and R³ have the same meanings as defined above (claim 19); the kit for an insoluble carrier particle nephelometric immunoassay according to claim 19, wherein the compound represented by general formula [II] is bicine or tricine (claim 20); the kit for an insoluble carrier particle nephelometric immunoassay according to any of claims 17 to 20, wherein the concentration of the buffer in the buffer solution is 5 to 200 mmol/L (claim 21); the kit for an insoluble carrier particle nephelometric immunoassay according to any of claims 17 to 21, wherein the concentration of insoluble carrier particles in the suspension is 0.005 to 5% by weight (claim 22); and the kit for an insoluble carrier particle nephelometric immunoassay according to any of claims 17 to 22, wherein the insoluble carrier particle is latex (claim 23).

### Best Mode of Carrying Out the Invention

There is no specific limitation as to an insoluble carrier particle nephelometric immunoassay reagent according to the present invention as long as the reagent contains insoluble carrier particles and a buffer comprising a compound having within its molecule a group represented by the following [Chemical formula 4] or a salt thereof. There is also no specific limitation as to a method of an insoluble carrier particle nephelometric immunoassay according to the present invention as long as the method comprises the use of insoluble carrier particles and a buffer comprising a compound having within its molecule a group represented by the following [Chemical formula 4] or a salt thereof. A method of an insoluble carrier particle nephelometric immunoassay mentioned here means a method of determining the amount of antigens or antibodies in samples by measuring turbidity generated by an immune agglutination reaction using insoluble carrier particles. Further, there is no specific limitation as to a kit for an insoluble carrier particle nephelometric immunoassay according to the present invention as long as the kit comprises a suspension containing insoluble carrier particles and a buffer solution containing a buffer comprising a compound having within its molecule a group represented by the following [Chemical formula 4] or a salt thereof.

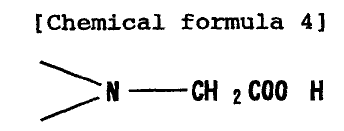

Examples of the above compounds as a buffer include a compound represented by following general formula [I], wherein R¹ and R² in general formula [I] may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxy-alkyl)alkyl group, and the like.

A linear or branched alkyl group having 1 to 6 carbon atoms is exemplified as an alkyl group in the aforementioned alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group and tri(sulfo-hydroxy-alkyl)alkyl group. Examples of such linear or branched alkyl groups having 1 to 6 carbon atoms include methyl group, ethyl group, propyl group, butyl group, isobutyl group, sec-butyl group, tert-butyl group, pentyl group, hexyl group, and the like.

Examples of substituents in the aforementioned substituted aminoalkyl group and substituted aminocarbonylalkyl group include alkyl group, hydroxyalkyl group, carboxyalkyl group, sulfoalkyl group, sulfo-hydroxyalkyl group, or nitrogen atom-containing heterocyclic groups. As an alkyl group in said alkyl group, hydroxyalkyl group, carboxyalkyl group, sulfoalkyl group and sulfo-hydroxy-alkyl group, the above-mentioned linear or branched alkyl group having 1 to 6 carbon atoms is exemplified. Examples of the nitrogen atom-containing heterocyclic groups include piperazinyl group, morpholino group, piperidino group, or the like.

Examples of substituents in the aforementioned substituted alkoxyalkyl group are hydroxyl group, carboxyl group and sulfone group, or the like.

Further, in general formula [I], R¹ and R² may form a cyclic structure together with a nitrogen atom to give substituted or unsubstituted piperazinyl group, substituted or unsubstituted morpholino group or substituted or unsubstituted piperidino group. Examples of substituents in said substituted piperazinyl group, substituted morpholino group and substituted piperidino group include alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxyalkyl)alkyl group, and the like.

As an alkyl group in the above-mentioned alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group and tri(sulfo-hydroxy-alkyl)alkyl group, the linear or branched alkyl group having 1 to 6 carbon atoms as mentioned above is exemplified.

As substituents in the substituted aminoalkyl group and substituted aminocarbonylalkyl group, the substituents in the substituted aminoalkyl group and substituted aminocarbonylalkyl group as mentioned above are exemplified. In addition, as the substituents in the substituted alkoxyalkyl group, the substituents in the substituted alkoxyalkyl group as mentioned above is exemplified.
[Chemical formula 5]

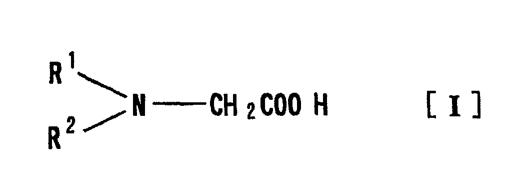

Examples of the hydroxyalkyl group include hydroxymethyl group, 2-hydroxyethyl group, 2-hydroxypropyl group, 2-hydroxybutyl group, 2-hydroxypentyl group, 2-hydroxyhexyl group, and the like; examples of the di(hydroxyalkyl)alkyl group include di(hydroxymethyl)methyl group, di(2-hydroxyetyl)methyl group, and the like; examples of the tri (hydroxyalkyl)alkyl group include tri (hydroxymethyl)methyl group, tri(2-hydroxyetyl)methyl group, and the like; examples of the carboxylalkyl group include carboxymethyl group, 2-carboxyethyl group, and the like; examples of the di(carboxylalkyl)alkyl group include di(carboxymethyl)methyl group, di(2-carboxyethyl)methyl group, and the like; and examples of the tri (carboxylalkyl)alkyl group include tri (carboxymethyl)methyl group, tri(2-carboxyethyl)methyl group, and the like.

Further, examples of the substituted or unsubstituted aminoalkyl group include aminomethyl group, 2-aminoethyl group, N-methylaminomethyl group, 2-(N-methylamino)ethyl group, and the like; examples of the substituted or unsubstituted aminocarbonylalkyl group include aminocarbonylmethyl group, 2- aminocarbonylethyl group, N-methylaminocarbonylmethyl group, 2-(N-methylaminocarbonyl)ethyl group, and the like; examples of the substituted or unsubstituted alkoxyalkyl group include methoxymethyl group, ethoxymethyl group, 2-methoxyethyl group, (carboxymethyloxy)methyl group, (2-hydroxyethyloxy)methyl group, and the like; examples of the sulfoalkyl group include sulfomethyl group, 2-sulfoethyl group, and the like; examples of the di(sulfoalkyl)alkyl group include di(sulfomethyl)methyl group, di(2-sulfoethyl)methyl group, and the like; examples of the tri(sulfoalkyl)alkyl group include tri(sulfomethyl)methyl group, tri(2-sulfoethyl)methyl group, and the like; examples of the sulfo-hydroxy-alkyl group include 2-hydroxy-3-sulfopropyl group, 3-hydroxy-4-sulfopropyl group, and the like; examples of the di(sulfo-hydroxy-alkyl)alkyl group include di(2-hydroxy-3-sufopropyl)methyl group, di(3-hydroxy-4-sulfopropyl)methyl group, and the like; and examples of the tri(sulfo-hydroxy-alkyl)alkyl group include tri(2-hydroxy-3-sulfopropyl)methyl group, tri(3-hydroxy-4-sulfopropyl)methyl group, and the like.

Still further, a compound represented by general formula [II] shown below is preferable among the compounds represented by general formula [I]. In general formula [II], R¹ , R² and R³ may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxyalkyl)alkyl group, and the like. An alkyl group in the alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group and tri(sulfo-hydroxy-alkyl)alkyl group are the same as mentioned above.

The substituents in the above-mentioned substituted aminoalkyl group, substituted aminocarbonylalkyl group and substituted alkoxyalkyl group are the same as mentioned above, and their respective examples are also the same as mentioned above.

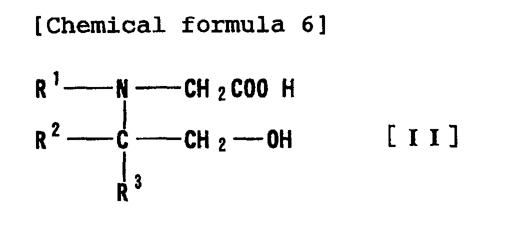

Examples of the buffer used in the present invention include bicine [N,N-bis(2-hydroxyethyl)glycine] and tricine {N-[tris(hydroxymethyl)methyl]glycine}, known as Good's buffer, as well as glycine, ADA [N-(2-acetamido)iminodiacetic acid], and the like. Among these, bicine represented by formula [III] and tricine represented by formula [IV] are preferable because they can stabilize more the insoluble carrier particle agglutination reaction.

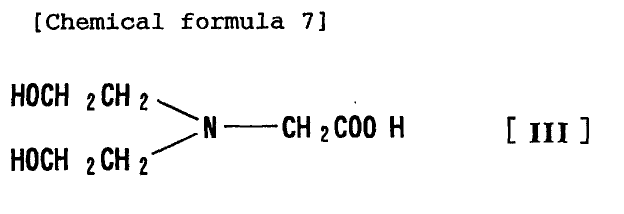

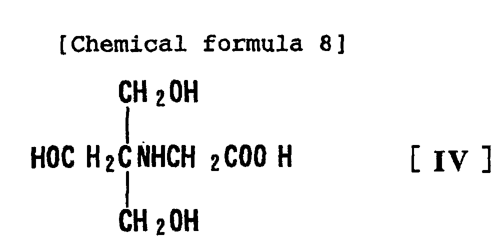

A buffer comprising a compound represented by general formula [I] mentioned above, especially a compound represented by general formula [II], is preferably used at the concentration range of 5 to 200 mmol/L because the agglutination reaction of insoluble carrier particles such as latex is more stabilized. In conducting an insoluble carrier particle agglutination reaction, the pH level during the reaction is very important to stabilize the reaction: pH can easily be kept at a certain level where the concentration of the buffer in the buffer solution is 5 mmol/L or higher; on the other hand, non-specific agglutination of insoluble carrier particles, which is not ascribed to antigen-antibody reaction, will not occur at a concentration of 200 mmol/L or lower. For adjusting the pH of a buffer solution containing a buffer, hydrochloric acid, sulfuric acid, nitric acid, or organic acids such as acetic acid may be used as acid; and sodium hydroxide, potassium hydroxide, lithium hydroxide, ammonium hydroxide, and the like, may be used as alkali. Further, the buffer solution of the present invention may include other optional components in addition to the above-mentioned components, if necessary. Examples of such optional components include surfactant effective for the solubilization of lipids in the samples, in particular, nonionic surfactant having a poly(oxyethylene)glycol group and the other, cationic or anionic surfactants.

As the insoluble carrier particles of the present invention, any kind of insoluble carrier particles may be used as long as the particles suppress the action of blood plasma components, which affect values to be determined, to stabilize the agglutination reaction when used along with the aforementioned buffer of the present invention. The examples include the known microparticles of an organic polymeric substance described in Japanese Published Examined Patent Application No. 11575/83, microparticles of inorganic oxides or microparticles wherein the surface of these substances that are to form the core is treated with an organic substance or the like. The specific examples are synthetic resin (latex) such as polystyrene, polyvinylchloride, polypropylene, (meta) acrylic resin, poly (methyl methacrylate); cellulose derivatives such as nitrocellulose, cellulose, methylcellulose; and inorganic substances such as metal, ceramics, glass, silicone rubber. Among these substances, particular preferable is a polystyrene synthetic polymer co-polymerized with an acrylate monomer or a monomer having sulfonic acid to provide electric charges.

As described above, latex particles, in particular, such as polystyrene latex or the like is preferably used in the present invention as the insoluble carrier mentioned above. Proteins and peptides can smoothly be adsorbed by using latex with a high surface hydrophobicity, such as polystyrene latex. In addition, polystyrene latex particles, obtained by the soap-free polymerization without a surfactant as an emulsifier, may preferably be used in particular, because they can remain stable without surfactants due to the repulsion raised between negative charges on the surface. Alternatively, various kinds of denatured latex (carboxylic acid denatured latex, for example), magnetic latex (latex containing magnetic particles) and the like may be used, if necessary.

As to insoluble carrier particles, equality of the size, controlling of the surface condition, selection of the internal structure and so on are usually required in a high level for conducting quantitative immunoassays, and favorable insoluble carrier particles for the preparation of reagents such as latex can be selected from those commercially available. Although the shape of an insoluble carrier particle is not limited to any particular shape, a sphere shape is exemplified. The preferable particle diameter is, for instance, 0.03 to 0.8 µm on average and especially 0.06 to 0.2 µm on average. In the present invention, although there is no particular limitation as to the concentration of insoluble carrier particles in the reaction solution, the concentration is, for example, 0.001 to 10% by weight, preferably 0.005 to 5% by weight and more preferably 0.01 to 2% by weight to stabilize the agglutination reaction of insoluble carrier particles more.

An example of a reagent for an insoluble carrier particle nephelometric immunoassay according to the present invention is a reagent comprising: a buffer such as bicine, tricine in such a manner that the applied concentration of the buffer can be adjusted to 5 to 200 mmol/L; insoluble carrier particles such as latex in such a manner that the applied concentration of the particles can be adjusted to 0.005 to 5% by weight; an antigen or an antibody to be carried on an insoluble carrier particle; and other optional components. Insoluble carrier particles having carried antigens or antibodies thereon in advance can also be comprised for said reagent.

Examples of a method of an insoluble carrier particle nephelometric immunoassay according to the present invention include: a method which comprises carrying an antigen or an antibody on an insoluble carrier particle by chemical bond, physical adsorption, or the like in the presence of a buffer such as bicine, tricine, and then conducting an immune agglutination reaction; and a method which comprises conducting an immune agglutination reaction using an insoluble carrier particle having carried antigens or antibodies thereon in advance in the presence of the buffer. In these methods, a buffer may be used as a buffer solution; and insoluble carrier particles as it is and insoluble carrier particles suspended in a buffer suspension may be used as an insoluble carrier particle suspension. It is preferable to have the buffer concentration at 5 to 200 mmol/L and the insoluble carrier particle concentration at 0.005 to 2% by weight during an immune agglutination reaction or when antigens or antibodies are carried by insoluble carrier particles.

Examples of a kit for the insoluble carrier particle nephelometric immunoassay according to the present invention include a kit which comprises: a buffer solution containing a buffer such as bicine, tricine at a concentration of 5 to 200 mmol/L; a suspension containing insoluble carrier particles such as latex at a concentration of 0.005 to 5% by weight; antigens or antibodies to be carried by insoluble carrier particles; and other optional components. Alternatively, insoluble carrier particles having carried antigens or antibodies thereon in advance may be comprised for said kit for the determination.

The present invention will be described in detail by the following examples, while the scope of the invention will not be limited to these examples. The % notations in the examples indicate % by weight unless otherwise stated.

### Example 1

### [Preparation of Reagent 1 (buffer solution)]

Bicine (DOJINDO Laboratories) (3.26 g) as a buffer was dissolved in distilled water, and then Triton X-100 (0.1 g), sodium chloride (17.5 g) and sodium azide (0.01 g) were added thereto. The pH was adjusted to 8.0 by adding 1 mol/L hydrochloric acid or sodium hydroxide solution while measuring the pH level at 20.degree. C. The solution was made 1000 mL in total by addition of distilled water. Further, instead of bicine, the following buffers were separately dissolved in distilled water: tricine (DOJINDO Laboratories) (3.58 g); TAPSO {2-hydroxy-3-[N-tris(hydroxymethyl)methylamino]propane sulfonic acid} (DOJINDO Laboratories) (5.18 g); POPSO [piperazine-1, 4-bis(2-hydroxy-3-propane sulfonic acid) · 2 hydrate] (DOJINDO Laboratories) (7.97 g); TES {2-(N-tris(hydroxymethyl)methylamino]ethanesulfonic acid}(DOJINDO Laboratories) (4.59 g). Triton X-100, sodium chloride and sodium azide were similarly added to each of the above solutions and their pHs were adjusted to 8.0, and then distilled water was added to the solutions to make each of the solutions 1000 mL in total. Thereby, the five kinds of buffer solutions were prepared: the two buffer solutions containing 20 mmol/L of bicine or tricine according to the present invention; and the three buffer solutions containing 20 mmol/L TAPSO, POPSO or TES for comparison.

### [Preparation of Reagent 2 (an antibody-carrying latex suspension)]

Latex was diluted by adding 9 equivalents of a 1/60 mol/L PBS solution (adjusted to pH 7.4 with 1 mol/L hydrochloric acid or sodium hydroxide solution) to one equivalent of 10% suspension of polystyrene latex (Kyowa Medex Co., LTD.) having the average particle diameter of 0.31 µm to give the 1% latex suspension. The anti-human ferritin antibody (Kyowa Medex Co., LTD.) was diluted with a 1/60 mol/L PBS solution to give the antibody solution containing the protein at a concentration of 50 µg/mL, with which the antibody was carried by latex. With stirring the 1% latex suspension (600 µL) with a magnetic stirrer at 25.degree. C. in an incubator, the antibody solution prerared above (1200 µL) was quickly added to the suspension, and the mixture was stirred at 25. degree. C. for two hours. Then, there was added a blocking solution (3 mL), which was prepared to contain BSA (Wako Pure Chemical Industries, Ltd.) by 0.6% and Triton X-100 (Sigma) by 0.015% in a 10 mmol/L glycine buffer solution, and the mixture was further stirred at 25.degree. C. for two hours. The mixture was then centrifuged at 15000 rpm, at 4.degree. C. for one hour. Subsequently, the obtained precipitate was washed by addition of the blocking solution (4 mL) followed by centrifugation of the mixture in a similar condition. Washing was conducted three times. The blocking solution (6 mL) was added to this precipitate to give the 0.1% antibody-carrying latex suspension.

### [Preparation of the samples]

Human blood was collected with a blood tube (VENOJECT Glass Vacuum Tubes; TERUMO Corp.) and was left for two hours to obtain a supernatant fluid (serum), which was made Sample 1. Human blood was further collected with an EDTA blood tube (VENOJECT Glass Vacuum Tubes; TERUMO Corp.)and was left for two hours to give a supernatant fluid (serum), which was made Sample 2. Furthermore, human blood was collected with a blood tube (VENOJECT Glass Vacuum Tubes; TERUMO Corp.) and was left for two hours to give a supernatant fluid (serum), and then this supernatant was supplemented with dipotassium ethylenediamine-tetraacetate (DOJINDO Laboratories) to 1 mg/mL, which was made Sample 3.

### [Diagram of a calibration curve using Reagents 1 and 2]

Ferritin (Scripps Laboratories Inc.) was dissolved in saline to prepare ferritin solutions with concentrations of 10.9, 21.9, 43.8, 87.5, 175 ng/mL, respectively. Each (10 µL) of these solutions was added to Reagent 1 (140 µL), and the reaction was allowed to occur at 37.degree. C. for 6 minutes and then Reagent 2 (150 µL) was added thereto. After the reaction at 37.degree. C. for 13 minutes, a calibration curve was diagramed by measuring changes in absorbance using Hitachi autoanalyzer 7170, by the 2 point-end method (photometric points: 21-39), with the main-wavelength of 750 nm and the sub-wavelength of 800 nm.

### [Determination of ferritin concentrations using Reagents 1 and 2]

In the same manner as in the diagram of the aforementioned calibration curve, each (10 µL) of the above-mentioned Samples 1,2 and 3 was added to Reagent 1 (140 µL) just after preparation, and the reactions were allowed to occur at 37.degree. C. for 6 minutes. Subsequently, Reagent 2 (150 µL) was added to each of the mixtures. After the reaction at 37.degree. C. for 13 minutes, changes in absorbance were measured using Hitachi autoanalyzer 7170, by the 2 point-end method (photometric points: 21-39), with the main-wavelength of 750 nm and the sub-wavelength of 800 nm, and the concentrations of ferritin in Samples were determined using the above-described calibration curve. The results are shown in Table 1. Table 2 shows the results determined in a similar way as described in the above except for using Reagent 1 of one-week after preparation instead of Reagent 1 just prepared. Tables 1 and 2 demonstrate that the determination sensitivity is stabilized when bicine or tricine is used as a buffer.

**Table 1**

| Buffer solution (just after preparation) | Ferritin concentration (ng/ml) | | |
|---|---|---|---|
| | Sample 1 | Sample 2 | Sample 3 |
| bicine | 40 | 39 | 39 |
| tricine | 41 | 40 | 41 |
| TAPSO | 39 | 30 | 38 |
| POPSO | 41 | 23 | 40 |
| TES | 41 | 31 | 40 |

**Table 2**

| Buffer solution (one week after preparation) | Ferritin concentration (ng/ml) | | |
|---|---|---|---|
| | Sample 1 | Sample 2 | Sample 3 |
| bicine | 40 | 39 | 39 |
| tricine | 41 | 40 | 41 |
| TAPSO | 41 | 37 | 41 |
| POPSO | 38 | 33 | 42 |
| TES | 42 | 38 | 42 |

### Example 2

### [Preparation of Reagent 3 (latex suspension)]

Bicine (3.26 g) as a buffer was dissolved in distilled water, and then 10% latex (particle diameter of 0.087 µm: SEKISUI CHEMICAL CO., LTD.) suspension (3.3mL) and sodium azide (0.1 g) were added thereto. While measuring the pH level at 20.degree. C., 1 mol/L sodium hydroxide solution or hydrochloric acid was added to the mixture to adjust the pH to 7.8. Distilled water was then added thereto to make the total amount 1000 mL. Instead of bicine, tricine (3.58 g), TAPSO (5.18 g), POPSO (7.97 g) and TES (4.59 g) as a buffer were separately dissolved in distilled water, and latex and sodium azide were added to each of the solutions, and the pH of each of the solutions was adjusted to 7.8 in a similar manner as before. Each solution was made 1000 mL by addition of distilled water. Thereby, the five kinds of buffer solutions were prepared: the two buffer solutions containing 20 mmol/L of bicine or tricine according to the present invention; and the three buffer solutions containing 20 mmol/L of TAPSO, POPSPO or TES for comparison.

### [Preparation of Reagent 4 (antibody solution)]

The anti-human HbAlc mouse monoclonal antibody, obtained in accordance with conventional method from mice immunized with a denatured human HbAlc as an antigen, was used for preparing the antibody solution. Bicine buffer (3.26 g) was dissolved in distilled water, and sodium chloride (15 g) was added thereto. Further, 1 mol/L hydrochloric acid or sodium hydroxide solution was added thereto while measuring the pH level at 20.degree. C. to adjust the pH to 7.0. Subsequently, Tween 20 (Wako Pure Chemical Industries, Ltd.) (2 g) and sodium azide (0.1 g) were added thereto. Furthermore, the above-mentioned anti-human denatured HbA1c mouse monoclonal antibody [0.025 g (in terms of IgG)] and anti-mouse IgG goat polyclonal antibody (Wako Pure Chemical Industries, Ltd.) [0.04 g (in terms of IgG)] were added thereto. Finally, the total amount was made 1000 mL by addition of distilled water to give the antibody solution. The antibody solutions were prepared in the same manner as described above except for using tricine (3.58 g), TAPSO buffer (5.18 g), POPSO (7.97 g) or TES buffer (4.59 g) instead of the bicine buffer. Each of the buffer solutions was dissolved in distilled water, and sodium chloride was added to these solutions, and the pH of each solution was adjusted to 7.0. In a similar manner as in the case of bicine, Tween 20 and sodium azide were added to each solution, and then an anti-human HbA1c mouse monoclonal antibody and an anti-mouse IgG goat polyclonal antibody were further added. The total amount of each of these solutions was made 1000 mL by addition of distilled water to give the antibody solutions.

### [Preparation of the samples]

Human blood was collected with an EDTA blood tube (VENOJECT Glass Vacuum Tubes; TERUMO Corp.). After leaving the blood for two hours, the precipitated blood cells phase (10 µL) was collected, which was then diluted with distilled water (1 mL) to prepare Sample 4. The blood plasma (10 µL), a supernatant fluid of the blood collected in the EDTA blood tube, was added to Sample 4 to prepare Sample 5 containing a blood plasma.

### [Diagram of a calibration curve using Reagents 3 and 4]

A calibration curve was diagramed by determining the changes in the absorbance with Hitachi autoanalyzer 7170 for each sample with the HbA1c levels of 0.0%, 4.2%, 7.7%, 11.3% and 14.8%, respectively. These HbAlc levels were determined by using TOSOH Automated Glycohemoglobin Analyzer HLC-723GHbV. The determination of the changes in the absorbance as mentioned above was carried out in the following steps: adding a sample (4 µL) to Reagent 3 (240 µL); allowing the reaction to proceed at 37. degree. C. for 5 minutes; adding Reagent 4 (80 µL) thereto; allowing the reaction to proceed at 37 degree. C. for 5 minutes; and determining the changes in absorbance by the 2 point-end method (photometric points: 16-34) at the main-wavelength of 450 nm and the sub-wavelength of 800 nm.

### [Determination of HbA1c concentrations using Reagents 3 and 4]

In a similar manner as in the above diagram of the calibration curve, each (4 µL) of Samples 4 and 5 mentioned above, was added to Reagent 3 (240 µL) just prepared, and the reaction was allowed to proceed at 37.degree. C. for 5 minutes. Then, Reagent 4 (80 µL) of three-day after preparation was added thereto, and the reaction was allowed to proceed at 37.degree. C. for 5 minutes. Changes in absorbance were measured with Hitachi autoanalyzer 7170 by the 2 point-end method (photometric points: 16-34) at the main-wavelength of 450 nm and the sub-wavelength of 800 nm. The HbAlc concentration of each sample was determined by using the above calibration curve. The results are shown in Table 3. In addition, Table 4 shows the results determined similarly as described above except that Reagent 3 of one-week after preparation was used instead of the just prepared Reagent 3. Tables 3 and 4 demonstrate that the determination sensitivity is stabilized when bicine or tricine is used as a buffer.

**Table 3**

| Buffer solution (just after preparation) | HbA1c level (%) | |
|---|---|---|
| | Sample 4 | Sample 5 |
| bicine | 6.1 | 6.1 |
| tricine | 6.1 | 6.0 |
| TAPSO | 6.1 | 5.4 |
| POPSO | 6.2 | 3.3 |
| TES | 6.1 | 5.3 |

**Table 4**

| Buffer solution (one week after preparation) | HbA1c level (%) | |
|---|---|---|
| | Sample 4 | Sample 5 |
| bicine | 6.1 | 6.1 |
| tricine | 6.1 | 6.0 |
| TAPSO | 6.2 | 5.6 |
| POPSO | 5.9 | 3.1 |
| TES | 6.3 | 5.6 |

### Industrial Applicability

According to the present invention, the action of blood plasma components, which are involved in the agglutination reaction of insoluble carrier particles and affect the values to be determined, is suppressed to stabilize the agglutination reaction as well as the absorbance of the reaction solution and to give accurate determination results.

## Claims

1. A reagent for an insoluble carrier particle nephelometric immunoassay containing insoluble carrier particles and a buffer comprising a compound within its molecule a group represented by [Chemical formula 1]. 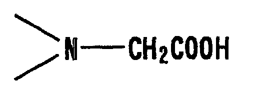 or a salt thereof.

2. The reagent for an insoluble carrier particle nephelometric immunoassay according to claim 1, wherein the compound is represented by general formula [I] 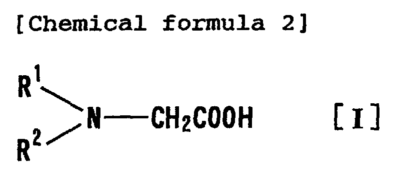 wherein R¹ and R² may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxy-alkyl)alkyl group, or R¹ and R² form a cyclic structure with a nitrogen atom to give substituted or unsubstituted piperazinyl group, substituted or unsubstituted morpholino group, or substituted or unsubstituted piperidino group.

3. The reagent for an insoluble carrier particle nephelometric immunoassay according to claim 2, wherein the compound represented by general formula [I] is a compound represented by general formula [II] 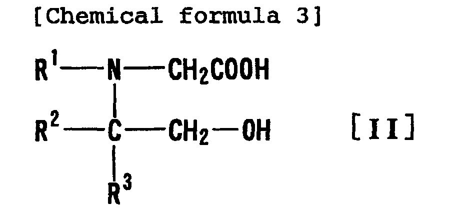 wherein R¹ , R² and R³ may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, or tri(sulfo-hydroxy-alkyl)alkyl group.

4. The reagent for an insoluble carrier particle nephelometric immunoassay according to claim 3, wherein the compound represented by general formula [II] is bicine or tricine.

5. The reagent for an insoluble carrier particle nephelometric immunoassay according to any of claims 1 to 4, wherein the buffer is contained in such a manner that the concentration can be adjusted to 5 to 200 mmol/L.

6. The reagent for an insoluble carrier particle nephelometric immunoassay according to any of claims 1 to 5, wherein the insoluble carrier particles are contained in such a manner that the concentration can be adjusted to 0.005 to 5% by weight.

7. The reagent for an insoluble carrier particle nephelometric immunoassay according to any of claims 1 to 6, wherein the insoluble carrier particle is latex.

8. A method of an insoluble carrier particle nephelometric immunoassay using insoluble carrier particles and a buffer comprising a compound having within its molecule a group represented by [Chemical formula 4] 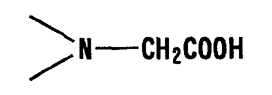 or a salt thereof.

9. The method of an insoluble carrier particle nephelometric immunoassay according to claim 8, wherein the compound is represented by general formula [I] 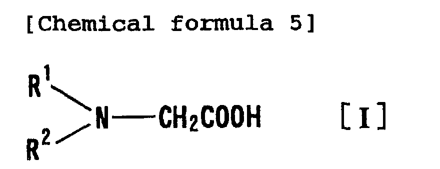 wherein R¹ and R² may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxy-alkyl)alkyl group, or R¹ and R² form a cyclic structure with a nitrogen atom to give substituted or unsubstituted piperazinyl group, substituted or unsubstituted morpholino group, or substituted or unsubstituted piperidino group.

10. The method of an insoluble carrier particle nephelometric immunoassay according to claim 9, wherein the compound represented by general formula [I] is a compound represented by general formula [II] 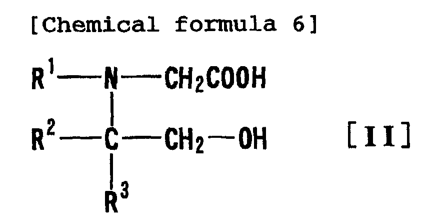 wherein R¹ , R² and R³ may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, or tri(sulfo-hydroxy-alkyl)alkyl group.

11. The method of an insoluble carrier particle nephelometric immunoassay according to claim 10, wherein bicine or tricine is used as the compound represented by general formula [II].

12. The method of an insoluble carrier particle nephelometric immunoassay according to any of claims 8 to 11, wherein an antigen or an antibody is carried on an insoluble carrier particle in the presence of a buffer, and then an immune agglutination reaction is allowed to proceed.

13. The method of an insoluble carrier particle nephelometric immunoassay according to any of claims 8 to 12, wherein an antigen or an antibody is carried on an insoluble carrier particle, and then an immune agglutination reaction is allowed to proceed in the presence of a buffer.

14. The method of an insoluble carrier particle nephelometric immunoassay according to any of claims 8 to 13, wherein the buffer is used in a buffer solution at a concentration of 5 to 200 mmol/L.

15. The method of an insoluble carrier particle nephelometric immunoassay according to any of claims 8 to 14, wherein insoluble carrier particles are used in an insoluble carrier particle suspension at a concentration of 0.005 to 5% by weight.

16. The method of an insoluble carrier particle nephelometric immunoassay according to any of claims 8 to 15, wherein the insoluble carrier particle is latex.

17. A kit for an insoluble carrier particle nephelometric immunoassay comprising a suspension containing insoluble carrier particles and a buffer solution containing a buffer comprising a compound having within its molecule a group represented by [Chemical formula 7] 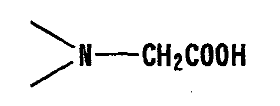 or a salt thereof.

18. The kit for an insoluble carrier particle nephelometric immunoassay according to claim 17, wherein the compound is represented by general formula [I] 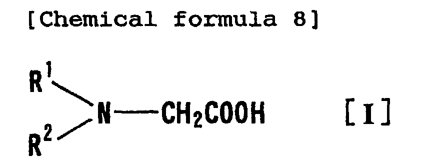 wherein R¹ and R² may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, tri(sulfo-hydroxy-alkyl)alkyl group, or R¹ and R² form a cyclic structure with a nitrogen atom to give substituted or unsubstituted piperazinyl group, substituted or unsubstituted morpholino group, or substituted or unsubstituted piperidino group.

19. The kit for an insoluble carrier particle nephelometric immunoassay according to claim 18, wherein the compound represented by general formula [I] is a compound represented by general formula [II] 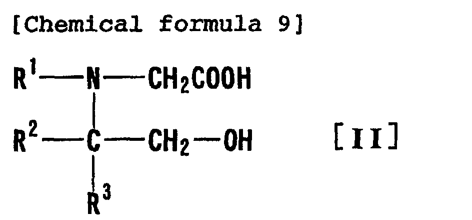 wherein R¹ , R² and R³ may be the same or different, and independently represent hydrogen atom, alkyl group, hydroxyalkyl group, di(hydroxyalkyl)alkyl group, tri(hydroxyalkyl)alkyl group, carboxyalkyl group, di(carboxyalkyl)alkyl group, tri(carboxyalkyl)alkyl group, substituted or unsubstituted aminoalkyl group, substituted or unsubstituted aminocarbonylalkyl group, substituted or unsubstituted alkoxyalkyl group, sulfoalkyl group, di(sulfoalkyl)alkyl group, tri(sulfoalkyl)alkyl group, sulfo-hydroxy-alkyl group, di(sulfo-hydroxy-alkyl)alkyl group, or tri(sulfo-hydroxy-alkyl)alkyl group.

20. The kit for an insoluble carrier particle nephelometric immunoassay according to claim 19, wherein the compound represented by general formula [II] is bicine or tricine.

21. The kit for an insoluble carrier particle nephelometric immunoassay according to any of claims 17 to 20, wherein the concentration of the buffer in the buffer solution is 5 to 200 mmol/L.

22. The kit for an insoluble carrier particle nephelometric immunoassay according to any of claims 17 to 21, wherein the concentration of insoluble carrier particles in the suspension is 0.005 to 5% by weight.

23. The kit for an insoluble carrier particle nephelometric immunoassay according to any of claims 17 to 22, wherein the insoluble carrier particle is latex.
